# EUROPÄISCHE PATENTSCHRIFT

(11) **EP 3 018 531 B1**
(45) Veröffentlichungstag und Bekanntmachung des Hinweises auf die Patenterteilung: **29.07.2020**
(21) Anmeldenummer: 14192461.3
(22) Anmeldetag: 10.11.2014
(51) Int. Cl.: G03F 7/00, A61L 27/36, A61F 2/06, B29C 67/00, C12N 5/00

(54) **Verfahren und Vorrichtung zur Erzeugung eines dreidimensionalen mehrzelligen Objektes**
Method and device for creating a three-dimensional multi-cell object
Procédé et dispositif de production d'un objet multi-cellulaires en trois dimensions

(43) Veröffentlichungstag der Anmeldung: 11.05.2016
(73) Patentinhaber: Technische Universität Berlin, 10623 Berlin (DE)
(72) Erfinder: Kloke, Lutz, 10245 Berlin (DE)
(74) Vertreter: Maikowski & Ninnemann Patentanwälte Partnerschaft mbB

(56) Entgegenhaltungen:
- EP-A2- 1 935 620
- WO-A1-2010/045951
- WO-A1-2013/158508
- WO-A2-2014/126830

## Beschreibung

Die vorliegende Erfindung betrifft ein Verfahren zur Erzeugung eines dreidimensionalen mehrzelligen Objektes gemäß dem Oberbegriff des Anspruchs 1, eine Vorrichtung zur Erzeugung eines dreidimensionalen mehrzelligen Objektes gemäß dem Oberbegriff des Anspruchs 12 sowie die Verwendung einer derartigen Vorrichtung gemäß dem Oberbegriff des Anspruchs 14.

Dreidimensionale mehrzellige Objekte können auch als zelluläre Objekte aus biologischen Material bezeichnet werden. Es ist bekannt, derartige Objekte durch Biodruckverfahren (sogenanntes Bioprinting) herzustellen, wie z.B. in der WO 2014/126830 A1 beschrieben. Dabei bezeichnet der Begriff "Drucken" das dreidimensionale Strukturieren von biologischen Material. Üblicherweise werden biologische Zellen mit Hilfe eines Gels in eine vorher gewählte Struktur geformt. Die Techniken, die für derartige Biodruckverfahren zum Einsatz kommen, sind das Tintenstrahldrucken (sogenanntes injekt printing), das Spritzendrucken (sogenanntes syringe printing oder bioplotting) und das Laserdrucken (sogenanntes laser printing). Jede dieser drei Techniken hat spezifische Vorteile, aber auch Limitierungen.

Beim Tintenstrahldruckverfahren werden Zellen in einer Flüssigkeit mit Hilfe einer Piezodüse auf einen Träger geschossen. Diese Technik funktioniert analog zur handelsüblichen Papier-Tintenstrahldrucktechnik, nur dass anstelle von Tinte eine biologische Tinte in Form einer Trägerflüssigkeit mit darin suspendierten Zellen zum Drucken eingesetzt wird. Mittels dieser Technik können sehr feine Mengen gedruckt werden, allerdings lässt die Genauigkeit des Verfahrens zu wünschen übrig, da die mittels der Piezodüsen erzeugten Tropfen mit biologischen Zellen in der Trägerflüssigkeit aus der Piezodüse geschossen werden und die Strecke zwischen dem Ende der Piezodüse und der zur Aufnahme des Druckobjekts bestimmten Oberflächen durch die Luft fliegen müssen. Während des Fluges kommt es zur Verformungen der Tropfen, wodurch die Tropfen Taumelbewegungen während des Fluges ausführen. Dies führt zu Ungenauigkeiten eines derartigen Tintenstrahldruckers, da die Zellen nicht immer den für sie vorgesehenen Ort erreichen. Ferner ist ein dreidimensionaler Schichtaufbau des zu erzeugenden Objektes limitiert, da nur von oben geschichtet werden kann, so dass Stützstrukturen und überhängende Strukturen schwierig herzustellen sind.

Derartige Tintenstrahldruckverfahren sind beispielsweise in der WO 99/48541 A1, der US 2009/0208466 A1, der US 2011/0076734 A1 und der US 2011/0250688 A1 beschrieben.

Das Spritzendruckverfahren ist das aktuell gängigste Druckverfahren im Biodruckbereich. Hier wird das zu druckende Material in eine Spritze geladen und durch Druckluft oder durch einen Stempeldruck aus der Spritze gedrückt. Die Düse der Spritze wird dabei von einer x-y-z-Bewegungseinheit in Abhängigkeit des zu druckenden Objektes an die für sie vorgesehene Stelle bewegt. An der für den Druck vorgesehenen Stelle wird dann eine definierte Menge des Druckmaterials aus der Spritze herausgedrückt. Auf diese Weise entsteht ein aus Schichten bestehendes dreidimensionales Objekt. Der Vorteil dieses Verfahrens ist ein einfacher Aufbau, allerdings kann nur mit hohem Aufwand genau dosiert werden. Sollen zudem unterschiedliche Zellen zum Aufbau des dreidimensionalen Objektes eingesetzt werden, müssen weitere Spritzen vorgehalten werden und zusätzlich zu der ersten Spritze oder anstelle der ersten Spritze für den Druck eingesetzt werden. Dies erhöht sowohl den konstruktiven Aufwand eines entsprechenden Druckers als auch die Zeit, die für den eigentlichen Druck erforderlich ist. Dies schlägt sich letztlich in hohen Kosten nieder.

Derartige Spritzendruckverfahren sind beispielsweise in der WO 2013/113883 A1, der US 2011/0313542 A1, der US 8,580,546 B2 und der US 2012/0288938 A1 beschrieben. Auch die US 2014/0093932 A1 beschreibt ein Spritzendruckverfahren, wobei hier zusätzlich eine Aushärtung des bereits an der beabsichtigten Stelle abgelagerten biologischen Materials mittels UV-Licht erfolgt.

Beim Laserdruckverfahren wird mit Hilfe eines Laserstrahls gedruckt. Dabei wird zunächst eine Trägerfolie mit einer Flüssigkeit beschichtet, die Zellen enthält. Anschließend wird ein Laserstrahlpuls auf die beschichtete Trägerfolie gelenkt, wodurch ein Tropfen der Zellsuspension von der Trägerfolie geschossen wird. Die einzelnen Tropfen können dann durch geschicktes Stapeln - ähnlich wie beim Tintenstrahldruckverfahren - aufeinander gestapelt werden. Zwar kann die Menge der zu applizierenden Tropfen der Zellsuspension recht genau dosiert werden. Allerdings verformen sich die Tropfen während der Flugphase auf ihrem Weg zur Oberfläche, auf der das zu druckende Objekt erzeugt werden soll. Aufgrund der damit verbundenen Taumelbewegung resultiert wiederum eine Ungenauigkeit bei der Positionierung der Tropfen. Da die Tropfengröße insgesamt eher klein ist, ist das Laserdruckverfahren sehr langsam. Größere und komplexere Objekte lassen sich auf diese Weise nicht drucken. Zudem können keine hängenden Strukturen ohne Stützstrukturen erzeugt werden.

Der vorliegenden Erfindung liegt die Aufgabe zugrunde, ein Druckverfahren für ein dreidimensionales Objekt aus biologischen Material anzugeben, das die aus dem Stand der Technik bekannten Nachteile überwindet und insbesondere eine hochpräzise Objektstrukturierung sowie die einfache Verwendung unterschiedlicher Materialen beim Drucken des Objektes ermöglicht. Ferner soll eine entsprechende Vorrichtung bereitgestellt werden, mit der das Verfahren umgesetzt werden kann.

Diese Aufgabe wird mit einem Verfahren zur Erzeugung eines dreidimensionalen mehrzelligen Objektes mit den Merkmalen des Anspruchs 1 gelöst. Bei einem solchen Verfahren wird zunächst eine erste photopolymerisierbare Flüssigkeit in ein Reaktionsgefäß eingebracht. Anschließend wird eine erste Lichtstrahlung auf eine erste Fokusebene, die innerhalb eines mit der ersten Flüssigkeit gefüllten Bereiches des Reaktionsgefäßes liegt, fokussiert. Durch diese Lichtstrahlung wird dann eine erste polymerisierte Struktur in dem Reaktionsgefäß erzeugt. Die erste polymerisierte Struktur befindet sich dabei in einer ersten Schicht.

In weiteren Verfahrensschritten wird eine weitere photopolymerisierbare Flüssigkeit in das Reaktionsgefäß eingebracht, wobei die zuvor erzeugte polymerisierte Struktur zumindest teilweise mit der weiteren photopolymerisierbaren Flüssigkeit bedeckt ist. Vorzugsweise ist die zuvor erzeugte polymerisierte Struktur vollständig mit der weiteren photopolymerisierbaren Flüssigkeit bedeckt. Nun wird eine weitere Lichtstrahlung auf eine weitere Fokusebene fokussiert, die innerhalb eines mit der weiteren Flüssigkeit befüllten Bereiches des Reaktionsgefäßes liegt. Die weitere Fokusebene unterscheidet sich damit von der ersten Fokusebene zumindest in Bezug auf die bereits erzeugte polymerisierte Struktur bzw. in Bezug auf die Schicht dieser polymerisierten Struktur.

Nun wird durch die weitere Lichtstrahlung eine weitere polymerisierte Struktur in einer weiteren Schicht in dem Reaktionsgefäß erzeugt. Dabei ist die weitere polymerisierte Struktur unmittelbar auf der zuvor erzeugten polymerisierten Struktur angeordnet und mit dieser verbunden. Bei der Verbindung handelt es sich vorzugsweise um eine kovalente Verbindung. Grundsätzlich wären jedoch auch nicht-kovalente Verbindungen, beispielsweise auf der Grundlage physikalischer Wechselwirkungen denkbar.

Die vorgenannten Schritte des Einbringens einer weiteren photopolymerisierbaren Flüssigkeit, des Fokussierens einer weiteren Lichtstrahlung und des Erzeugens einer weiteren polymerisierten Struktur in einer weiteren Schicht werden nun mit jeweils einer weiteren photopolymerisierbaren Flüssigkeit wiederholt, bis das gewünschte dreidimensionale mehrzellige Objekt erzeugt ist. Durch die unterschiedlichen Fokusebenen, in denen eine Polymerisierung der photopolymerisierbaren Flüssigkeiten stattfindet, wird folglich ein schichtweiser Aufbau des dreidimensionalen mehrzelligen Objektes erreicht. Dabei sind auch Hinterschnitte und überhängende Strukturen möglich, da eine Polymerisierung der photopolymerisierbaren Flüssigkeit in einer bestimmten Fokusebene beziehungsweise Schicht auch dann erfolgen kann, wenn darunter kein bereits polymerisiertes Material angeordnet ist, sondern lediglich noch nicht polymerisierte Flüssigkeit. Eine Polymerisierung dieser in einer außerhalb der Fokusebene vorliegenden photopolymerisierbaren Flüssigkeit bleibt aus; vielmehr wird nur diejenige photopolymerisierbare Flüssigkeit polymerisiert, die innerhalb der Fokusebene liegt. Dennoch dient die außerhalb der Fokusebene vorhandene Flüssigkeit zur temporären Stützung der in der Fokusebene vorhandenen Flüssigkeit, ohne dass hierfür feste Stützstrukturen erforderlich wären.

Die erste photopolymerisierbare Flüssigkeit und/oder zumindest eine der weiteren photopolymerisierbaren Flüssigkeiten enthalten biologische Zellen. Wenn es in Folge der Lichteinstrahlung zu einer Polymerisierung kommt, werden die in der Flüssigkeit enthaltenen Zellen mit in ein entsprechendes Polymer eingebettet. Da nicht alle photopolymerisierbaren Flüssigkeiten auch Zellen enthalten müssen, können in dem erzeugten dreidimensionalen mehrzelligen Objekt zellfreie Strukturen ausgebildet werden, beispielsweise in Form intermediärer Strukturen.

Mit diesen Verfahren können komplexe biologische Objekte als Modelle erzeugt werden, um beispielsweise Zell-Zell-Interaktionen, die Organbiogenese, Krankheiten oder Organfunktionen darzustellen und zu untersuchen. Ein derartiges dreidimensionales Objekt hat gegenüber der klassischen zweidimensionalen Zellkultur deutliche Vorteile, insbesondere wenn es um die Modellierung des Zusammenspiels mehrerer Zelltypen kommt. Denn die Komplexität von Zell-Zell-Interaktionen, die Funktion einer natürlichen Barriere und die Modulierung von Krankheiten oder Organen kann mit den klassischen zweidimensionalen Zellkulturen nicht hinreichend abgebildet werden.

Darüber hinaus ermöglicht das vorliegend beschriebene Verfahren die Erstellung miniaturisierter Modelle in besonders einfacher Art und Weise. Derartige miniaturisierte Modelle wurden bislang teilweise von Hand aufgebaut. Der hierfür erforderliche Aufwand ist sehr hoch; zudem ist langjährige Erfahrung notwendig.

Schließlich kann durch das vorliegend beschriebene Verfahren eine hohe Reproduzierbarkeit unterschiedlicher Exemplare des gleichen dreidimensionalen mehrzelligen Objektes gewährleistet werden. Folglich lässt sich durch das vorliegend beschriebene Verfahren nicht nur die Herstellung gegenüber anderen, aus dem Stand der Technik bekannten Verfahren beschleunigen, vielmehr weisen die erzeugten Objekte auch stets die gleiche Qualität auf. Eine derartige hohe Reproduzierbarkeit ist in der Biotechnologie besonders vorteilhaft. Denn bei der Analyse und Entwicklung neuer pharmazeutischer Produkte reduziert ein Test an stets gleichbleibenden dreidimensionalen Zellkulturen die Entwicklungskosten erheblich. Wenn derart komplexe dreidimensionale Strukturen hingegen per Hand aufgebaut werden, sind individuelle Schwankungen unvermeidlich. Damit wird es aber praktisch unmöglich, reproduzierbare Testergebnisse zu erhalten. Demgegenüber liefert das vorliegend beschriebene Verfahren Objekte, die für die Erzeugung reproduzierbarer Testergebnisse hervorragend geeignet sind.

Als Reaktionsgefäße können Kavitäten (sogenannte wells) handelsüblicher Mikrotiterplatten (beispielsweise Mikrotiterplatten mit 6, 12, 24, 48, 96, 384 oder 1536 Kavitäten), Zellkulturflaschen oder Petrischalen verwendet werden.

Das mittels des Verfahrens hergestellte dreidimensionale mehrzellige Objekt kann aus einem homogenen Material aufgebaut sein und folglich nur Zellen eines einzigen Typs umfassen. Ferner kann das die Zellen umgebende Polymermaterial einheitlich ausgestaltet sein. In einer Variante handelt es sich bei der ersten photopolymerisierbaren Flüssigkeit und bei zumindest einer der weiteren photopolymerisierbaren Flüssigkeiten jedoch um unterschiedliche Flüssigkeiten. Dadurch wird es möglich, heterogen aufgebaute mehrzellige Objekte zu erzeugen, die Zellen unterschiedlichen Typs umfassen. Ferner ist es auf diese Art und Weise möglich, gleiche oder unterschiedliche Zellen in unterschiedlichen umhüllenden Polymeren bereitzustellen. Das heißt, die unterschiedlichen Flüssigkeiten können sich sowohl hinsichtlich des polymerisierbaren Materials in der Flüssigkeit als auch hinsichtlich der in der Flüssigkeit enthaltenen Zellen unterscheiden. Mit dieser Variante wird folglich ein Verfahren bereitgestellt, mit dem unterschiedliche Zelltypen zu einem künstlichen Organ zusammengesetzt werden. Bei diesem künstlichen Organ kann es sich um ein Organ handeln, dass ein menschliches oder ein tierisches Organ nachbildet beziehungsweise imitiert. Dabei ist es möglich, innerhalb eines einzigen Druckvorganges unterschiedliche Objekte innerhalb desselben Reaktionsgefäßes oder innerhalb benachbarter Reaktionsgefäße zu erzeugen.

Wenn mittels des vorliegenden Verfahrens ein künstliches Organ erzeugt wird, kann es sich bei diesem Organmodell um ein Modell handeln, das ein gesundes Organ nachbildet. Alternativ kann auch ein Krankheitsmodell in Form eines einen bestimmten Defekt aufweisenden Organs erzeugt werden. Beispielsweise kann eine mechanische Verengung in ein mit dem vorliegenden Verfahren erzeugtes Objekt eingebracht werden, um eine "eingebaute" Verletzung wie beispielsweise bei einem stumpfen Schlag oder einer stumpfen Verletzung zu erzeugen. Auf diese Weise ist es möglich, einen standardisierten Aufbau für derartige Krankheitsmodelle bereitzustellen. Ebenso ist es möglich, innerhalb eines Objektes einen degenerativen Gradienten zu erzeugen. So kann in einem ersten Bereich des Objektes eine verhältnismäßig gesunde Zellstruktur erzeugt werden, die dann kontinuierlich über einen örtlichen Gradienten in dem erzeugten Objekt zu einer kranken Struktur überführt wird. Zwischen den beiden extremen Strukturen würden sich dann intermediäre, teilweise kranke Strukturen in dem erzeugten Objekt befinden. Darüber hinaus ist es auch möglich, durch den Einbau von Viren oder Bakterien Infektionsmodelle für künstliche Organe zu erzeugen. Ein derartiger Einbau von Bakterien oder Viren kann in der oben beschriebenen Art und Weise während des Druckvorgangs durch die Auswahl einer geeigneten weiteren photopolymerisierbaren Flüssigkeit, die entsprechende Viren oder Bakterien als Zellen enthält, erfolgen.

Durch die Auswahl geeigneter Zelltypen lassen sich darüber hinaus Krankheitsmodelle für typische Erkrankungen wie etwa Diabetes, Tumore oder beeinträchtigtes Gewebe, wie es nach einem Herzinfarkt oder einem Schlaganfall in einem lebendenden Organismus vorgefunden wird, erzeugen. Da neben zellhaltigen photopolymerisierbaren Flüssigkeiten auch zellfreie photopolymerisierbare Flüssigkeiten eingesetzt werden können, lässt sich eine "Verpackung" des erzeugten Objektes in einem zellfreien Polymer im selben Druckvorgang erzeugen, in dem auch das Objekt selbst erzeugt wird. Das heißt, das Objekt wird zeitgleich mit und in seinem Träger generiert, so dass die Verpackung und das mehrzelligen Objekt parallel entstehen.

In einer Variante weist die photopolymerisierbare Flüssigkeit eine Acrylverbindung auf, mittels derer die Polymerisierung bewerkstelligt wird. Die Acrylverbindung ist vorzugsweise ausgewählt aus der Gruppe bestehend aus Methacylaten, Methylacrylaten, Ethylacrylaten, Hydroxyethylacrylaten, Butylacrylaten, Trimethylolpropanacrylaten, Triacrylatacrylaten und Polyacrylaten (PA) im allgemeinen.

Die Acrylverbindung wird in einer Variante an eine zu gelierende bzw. zu polymerisierende Ausgangssubstanz gekoppelt. Durch diese Kopplung entsteht insbesondere eine kovalente Verbindung zwischen der Acrylverbindung und der zu polymerisierenden Ausgangssubstanz. Bei dieser Ausgangssubstanz kann es sich beispielsweise um ein Polymer auf Kohlenstoffbasis, wie etwa Polyethylenglykol (PEG), Polyethylen (PE), Polypropylen (PP), Polyketon (PK), Polyvinylchlorid (PVC), Polystyrol (PS), Polytetrafluorethylen (PTFE), Polymethylmethacrylat (PMMA), Polycarbonat (PC), Polyethylenterephthalat (PET) und Polyurethan (PU) handeln. Ferner sind synthetische Polymere wie Silikone, Polydimethylsiloxan (PDMS) oder Harze wie etwa Melamin- oder Melamin-FormaldehydHarze als Ausgangssubstanz geeignet. Ferner sind Biopolymere wie etwa Proteine, DNA, RNA, Kohlenhydrate und Kohlenhydratderivate, Kollagene, Fibrine, Alginate oder Gelantine als Ausgangssubstanzen geeignet. Statt der vorgenannten Polymere können auch jeweils die Monomervorstufen oder Oligomervorstufen dieser Polymere als Ausgangssubstanzen eingesetzt werden, sofern diese in stabiler Weise im festen oder flüssigen Aggregatzustand bereitgestellt werden können. Durch das Einbringen einer Acrylatfunktion in die Ausgangssubstanz durch die Kopplung zwischen der Acrylverbindung und der Ausgangssubstanz wird eine polymerisierbare Matrix bereitgestellt, auch wenn es sich bei den Ausgangssubstanzen bereits um Polymere handelt.

Wenn photopolymerisierbares PDMS als Matrix bzw. als Hüllsubstanz eingesetzt wird, ist ein Gasaustausch zwischen den in dieser Matrix eingebetteten Zellen möglich. Wie bereits erwähnt, können unterschiedliche Hüllsubstanzen bzw. Matrices eingesetzt werden. So kann beispielsweise neben PDMS oder einer anderen Matrix, die eine gute Biokompatibilität aufweist, ein stabiler Kunststoff für die restliche Matrix eingesetzt werden, um so ein nach außen stabiles Objekt, in dessen Inneren eine das Zellwachstum ermöglichende Matrix mit geringerer Stabilität vorliegt, zu erzeugen. Wie bereits erwähnt, kann man also sagen, dass parallel zum dreidimensionalen mehrzelligen Objekt sein eigener Schutz bzw. seine eigene Verpackung erzeugt werden kann.

Die um die Acrylatfunktion ergänzte Ausgangssubstanz wird in flüssiger Weise eingesetzt, wobei unterschiedliche Viskositäten möglich sind. Das heißt, das vorliegend beschriebene Verfahren ist nicht auf Flüssigkeiten mit einer bestimmten Viskosität beschränkt, sondern kann auch niedrigviskose Flüssigkeiten als Ausgangssubstanzen einsetzen. Dabei kann das Fließverhalten dieser Flüssigkeiten von Thixotropie bis Rheopexie reichen.

Die Flüssigkeiten können Lösungen oder kolloid-disperse Gemische, wie etwa Suspensionen, sein. Die Flüssigkeiten können dabei einen wässrigen bis öligen Charakter haben. Dies wird u.a. durch die Wahl der Ausgangssubstanzen und deren Partikelgröße bestimmt.

Damit eine Photopolymerisierung der eine Acrylatfunktion tragenden Ausgangssubstanz erreicht werden kann, wird zudem ein Radikalbildner (ein sogenannter Photoinitiator) eingesetzt, der bei einer ausgewählten Wellenlänge des im Rahmen des Verfahrens eingesetzten Lichtes Radikale bildet.

Geeignete Radikalbildner sind beispielsweise Anthronderivate wie etwa Violanthron oder Isoviolanthron, Fluoreszein, Rubren, Anthrazinderivate, Tetrazenderivate, Benzanthron, Benzanthronil, Eosin, Levolinsäurederivate, Phospin-Derivate, Mono- und Bis-Acyl-Phosphine, Metallocene, Acetophenone, Benzophenone, Xanthone, Quinone, Keton-Derivate, Hydroxyketone, Aminoketone, Benzoyl-Peroxide, Pyridin-Salze, Phenylglyoxylate und/oder Iodonium-Salze.

Vorzugsweise werden zusätzlich zu dem Radikalbildner auch noch ein Vinylmakromer und ein Amin-basierter Co-Initiator eingesetzt, um die Photopolymerisation in besonders geeigneter Weise ablaufen zu lassen. Als Co-Initiator sind beispielsweise Ascorbinsäure und tetiäre Aminderivate, wie etwa Methyldiethanolamin oder Tetraethylamin geeignet.

In einer Variante weist die photopolymerisierbare Flüssigkeit ein Thiolderivat auf. Geeignete Thiolderivate sind Dithiothreitol, monofunktionale Cysteine, bifunktionale Peptide und ähnliche Verbindungen.

Darüber hinaus kann der photopolymerisierbaren Flüssigkeit eine Substanz zugesetzt werden, die eine Photopolymerisation tieferer, flüssiger Schichten verhindert. So bleibt flüssige Lösung außerhalb der Fokusebene flüssig, auch wenn sie sich im Einstrahlbereich der über ihr liegenden Fokusebene befindet. Dies funktioniert durch eine Absorption der polymerisierenden Wellenlänge durch die Substanz. Das Abfangen findet in der Fokusebene statt, so dass kein Eindringen der polymerisierenden Wellenlänge in tiefere Schichten möglich ist. Geeignet sind alle Substanzen, die in der gewünschten Wellenlänge absorbieren, wie etwa Farbstoffe.

In einer weiteren Variante weist die photopolymerisierbare Flüssigkeit ein monofunktionales Monomer auf, wie etwa N-Vinylpyrrolidon.

Darüber hinaus ist es in einer Variante möglich, dass die erste photopolymerisierbare Flüssigkeit und/oder eine der weiteren photopolymerisierbaren Flüssigkeiten und/oder eine andere Flüssigkeit, die nicht photopolymerisierbar sein muss, einen temperatursensitiven Gelbildner aufweisen. Insbesondere ist der Einsatz eines invers-temperatursensitiven (auch als revers-temperatursensitiv bezeichneten) Gelbildners vorgesehen. Ein solcher Gelbildner wird mit steigender Temperatur fester. Durch ein Erwärmen des Reaktionsgefäßes erstarrt die Reaktionsflüssigkeit und bildet ein zunächst nur metastabiles Gel. Sollte die Flüssigkeit nicht gleichzeitig photopolymerisiert werden, kann durch ein nachfolgendes Abkühlen des Objekts das metastabile Gel wieder verflüssigt und abgepumpt werden. Bei gewöhnlichen temperatursensitiven Gelbildnern liegen die anzuwendenden Temperaturverhältnisse genau umgekehrt. So kann bei Bedarf beispielsweise eine Stützstruktur erstellt werden, so dass hängende Strukturen erzeugbar sind. Wird das metastabile Gel hingegen zumindest teilweise mit Licht in geeigneter Wellenlänge bestrahlt, kommt es zu einer Photopolymerisierung, so dass das metastabile Gel in diesen Bereichen in ein stabiles Gel bzw. Polymer umgesetzt wird.

Mit anderen Worten ausgedrückt, kann durch den temperatursensitiven, insbesondere invers-temperatursensitiven, Gelbildner und eine Temperaturkontrolle des Reaktionsraumes noch einfacher mit hängenden Partien und Hinterschnitten gearbeitet werden. Es kann auch in dieser Variante aber weiterhin auch mit flüssigen Strukturen als Stütze gearbeitet werden.

Es ist darüber hinaus möglich, einen Temperaturgradienten vorzusehen, so dass ein metastabiles Gel nicht in allen Bereichen der mit dem temperatursensitiven, insbesondere invers-temperatursensitiven, Gelbildner versetzten Flüssigkeit erfolgt. Mit Hilfe eines solchen Gradienten können noch komplexere Strukturen erzeugt werden.

Die vorgenannten einzelnen Komponenten können als individuelle Substanzen in der photopolymerisierbaren Flüssigkeit enthalten sein. Alternativ ist es auch möglich, die zur Gelbildung vorzugsweise eingesetzten Substanzen bzw. Gruppen in einem einzigen Polymer durch entsprechende Synthese zu realisieren. Statt einer Mischung aus einzelnen Komponenten würde ein derartiges Polymer dann unterschiedliche funktionelle Gruppen aufweisen, die alle für eine Photopolymerisation benötigten bzw. vorzugsweise einzusetzenden Funktionen in sich vereinen. Ferner ist es auch denkbar, lediglich einige der für die Photopolymerisation vorzugsweise eingesetzten Funktionen bzw. Gruppen in einem Polymer vorzusehen und andere für die Photopolymerisation vorzugsweise einzusetzende Funktionen bzw. Gruppen in separaten Einzelkomponenten der photopolymerisierbaren Flüssigkeit zuzumischen.

Als biologische Zellen, die für den Aufbau des dreidimensionalen mehrzelligen Objektes eingesetzt werden, kommen sämtliche natürlich vorkommenden eukaryotischen und prokaryotischen Zellen in Betracht. Vorzugsweise handelt es sich bei den eingesetzten Zellen um eukaryotische Zellen. Besonders geeignet sind alle Zellen und Zelltypen, die im Körper eines Säugetiers, insbesondere eines Nagers und ganz besonders eines Menschen, vorkommen bzw. diesen Körper aufbauen. In einer Variante handelt es sich bei den eingesetzten biologischen Zellen um omnipotente oder pluripotente Zellen. Dabei bezieht sich die Erfindung in einer Variante nur auf den Einsatz solcher Zellen, die ohne die Zerstörung menschlicher Embryonen gewonnen werden können. Neben natürlich vorkommenden Zellen können als biologische Zellen auch Zellen nicht natürlich vorkommender Zelllinien verwendet werden. Derartige künstlich generierte Zelllinien ermöglichen den maßgeschneiderten Aufbau des herzustellenden dreidimensionalen mehrzelligen Objektes.

Da das vorliegende Verfahren eine Kombination verschiedener Zelltypen zu einem dreidimensionalen mehrzelligen Objekt ermöglicht, eignet es sich insbesondere zur Herstellung künstlicher Organe. Derartige künstliche Organe können beispielsweise miniaturisierte Modellobjekte eines natürlich vorkommenden Organs, insbesondere eines natürlich vorkommenden Organs eines Menschen oder eines Tieres, wie etwa eines Säugetieres oder eines Nagers sein. Da unterschiedliche photopolymerisierbare Flüssigkeiten eingesetzt werden können, sind auch unterschiedliche Geltypen, in denen die biologischen Zellen eingebettet sind, möglich. Zudem ist es möglich, Kunststoffpolymere und Biopolymere zu kombinieren, so dass sehr stabile Gebilde hergestellt werden können, in denen die biologischen Zellen eingebettet sind. Während eines einzelnen Druckvorganges können mehrere dreidimensionale Objekte, auch mit unterschiedlichen Formen, gleichzeitig erzeugt werden.

Des Weiteren lässt sich durch die Membran- und Barrierefunktion in Kombination mit Organfunktion ebenfalls das Modell einer Schwangerschaft durch diese Technologie simulieren.

In einer Variante handelt es sich bei den erzeugten künstlichen Organen insbesondere um solche Organe, die die Funktion von Muskeln, des Skeletts, der Haut, des Fettgewebes, des Darms, der Leber, des Knochenmarks, des Gehirns, der Lunge, des Herzens, der Niere, der Schilddrüse oder der Milz nachbilden und folglich als künstliche Muskeln, künstliches Skelett etc. bezeichnet werden können.

Auf einem Träger innerhalb eines Reaktionsgefäßes oder auch in unterschiedlichen Reaktionsgefäßen können Kombinationen verschiedener künstlicher Organe, die beispielsweise während desselben Druckvorgangs erzeugt wurden, bereitgestellt werden.

In einer Variante wird die weitere photopolymerisierbare Flüssigkeit erst dann in das Reaktionsgefäß eingebracht, wenn die zuvor im Reaktionsgefäß befindliche photopolymerisierbare Flüssigkeit (dies kann beispielsweise die erste photopolymerisierbare Flüssigkeit oder eine weitere photopolymerisierbare Flüssigkeit sein) aus dem Reaktionsgefäß entfernt wurde. Hierzu ist es beispielsweise möglich, dass eine Pumpe vorgesehen ist, welche eine bereits verwendete photopolymerisierbare Flüssigkeit aus dem Reaktionsgefäß herauspumpt und eine neue weitere photopolymerisierbare Flüssigkeit in das Reaktionsgefäß hineinpumpt. Statt einer einzelnen Pumpe können für derartige Vorgänge auch zwei oder mehr unterschiedliche Pumpen verwendet werden.

Es ist vorgesehen, in einer Variante eine desinfizierend Flüssigkeit in das Reaktionsgefäß einzuleiten, um eine sterile Herstellung des dreidimensionalen mehrzelligen Objektes zu ermöglichen. Eine derartige desinfizierende Flüssigkeit kann beispielsweise in das Reaktionsgefäß dann eingebracht werden, wenn eine zuvor verwendete photopolymerisierbare Flüssigkeit aus dem Reaktionsgefäß entfernt wurde und eine weitere photopolymerisierbare Flüssigkeit noch nicht in das Reaktionsgefäß eingebracht wurde. Es ist darüber hinaus auch möglich, eine desinfizierende Flüssigkeit zusätzlich zu einer photopolymerisierbaren Flüssigkeit in das Reaktionsgefäß einzubringen, so dass sie während des Photopolymerisationsprozesses in dem Reaktionsgefäß enthalten ist.

Als desinfizierende Flüssigkeit kann beispielsweise ein Alkohol wie etwa Ethanol oder Propanol eingesetzt werden. Dabei sind insbesondere wässrige Lösungen derartiger Alkohole zur Desinfektion geeignet, wobei die Alkoholkonzentration beispielsweise im Bereich von 40 % bis 90 %, insbesondere von 50 % bis 80 % und ganz besonders von 60 % bis 70 % (jeweils v/v) liegt.

In einer Variante kann das mehrzellige dreidimensionale Objekt während oder am Ende seines Herstellungsprozesses mit Licht einer niedrigen Wellenlänge (beispielsweise im UV-Bereich, also unterhalb von 380 nm) bestrahlt werden, um auf diese Weise eine Sterilisation zu erreichen. Derartige UV-Sterilisationen sind grundsätzlich bekannt. Sie lassen sich im vorliegenden Fall jedoch dann sinnvoll einsetzen, wenn die biologischen Zellen, welche im dreidimensionalen Objekt enthalten sind, durch eine derartige UV-Strahlung nicht geschädigt werden.

In einer Variante ist eine Trägerplatte oder Trägerstruktur in dem Reaktionsgefäß angeordnet, an die die erste polymerisierte Struktur gebunden wird. Der Einsatz einer derartigen Trägerplatte bietet sich dann an, wenn das erzeugte dreidimensionale mehrzellige Objekt später nicht im Reaktionsgefäß selbst untersucht werden soll, sondern aus dem Reaktionsgefäß entnommen werden soll. In der Trägerplatte können beispielsweise Schraubanschlüsse (wie etwa DIN-Schraubanschlüsse) vorhanden sein, um eine nachfolgende Versorgung des erzeugten mehrzelligen dreidimensionalen Objektes mit Flüssigkeiten und Gasen zu ermöglichen. Es ist auch möglich, derartige Schraubanschlüsse in die Matrix des dreidimensionalen mehrzelligen Objektes im Rahmen des Herstellungsverfahrens einzubringen, diese Schraubanschlüsse also dort in der Matrix zu generieren. Das Erzeugen derartiger Schraubanschlüsse in der Matrix kann unabhängig davon vorgenommen werden, ob eine Trägerplatte eingesetzt wird oder nicht.

In einer Variante wird eine Trägerplatte vor dem Schritt des Erzeugens einer ersten polymerisierten Struktur durch das Einstrahlen einer Lichtstrahlung in eine Fokusebene, die innerhalb eines mit einer photopolymerisierbaren Flüssigkeit (insbesondere mit der ersten oder einer der weiteren photopolymerisierbaren Flüssigkeit) gefüllten Bereichs des Reaktionsgefäßes liegt, erzeugt, indem eine polymerisierte Trägerstruktur gebildet wird, die die Trägerplatte aufweist oder darstellt. Das heißt, in dieser Variante werden nicht nur die eigentlichen polymerisierten Strukturen, sondern auch die Trägerstruktur durch eine Polymerisierungsreaktion erzeugt.

Die Trägerstruktur kann eine solche Form haben, dass zwischen der Trägerplatte und einem Boden des Reaktionsgefäßes ein Abstand gebildet ist. Dadurch weisen dann die Fokusebenen der eigentlichen Polymerisierungsreaktionen einen größeren Abstand zum Boden des Reaktionsgefäßes auf. Insbesondere weist die erste gebildete polymerisierte Struktur dann einen größeren Abstand zum Boden des Reaktionsgefäßes auf. Dann lassen sich nicht mehr benötigte polymerisierbare Flüssigkeiten besonders leicht aus dem Reaktionsgefäß absaugen.

Damit die Flüssigkeiten einfach durch die Trägerplatte bzw. die Trägerstruktur hindurchtreten können, kann die Trägerstruktur mit flüssigkeitsdurchlässigen Durchbrüchen versehen sein, insbesondere im Bereich der Trägerplatte.

In einer Variante wird zwischen einer Lichtquelle, die zur Erzeugung der ersten und/oder der weiteren Lichtstrahlung dient, und dem Reaktionsgefäß ein optisches System angeordnet, das zur Fokussierung der Lichtstrahlung auf die jeweilige Fokusebene in dem Reaktionsgefäß dient. Dabei ist es in einer Variante vorgesehen, dass eine Refokussierung dieses optischen Systems erfolgen kann, um die Fokusebene innerhalb des Reaktionsgefäßes zu ändern. Eine derartige Refokussierung kann beispielsweise durch eine Veränderung des Abstands des optischen Systems zur Lichtquelle erreicht werden. Dabei kann ein computergesteuerter Schrittmotor vorgesehen sein, um eine entsprechende Bewegung des optischen Systems zu vermitteln. Beim optischen System kann es sich beispielsweise um ein System optischer Linsen oder - in einem konstruktiv besonders einfach gelagerten Fall - um eine einzelne Fokussierlinse handeln.

Wenn eine Refokussierung eines optischen Systems durchgeführt wird, um die Fokusebene innerhalb des Reaktionsgefäßes zu ändern bzw. zu verschieben, werden an die Ausgestaltung des Reaktionsgefäßes regelmäßig keine besonderen Anforderungen gestellt.

In einer Variante ist es auch möglich, eine Relativbewegung zwischen dem Reaktionsgefäß oder einer in dem Reaktionsgefäß angeordneten Trägerplatte auf der einen Seite und einer Lichtquelle, die zur Erzeugung der ersten und/oder der weiteren Lichtstrahlung dient, auf der anderen Seite durchzuführen. Denn durch eine derartige Relativbewegung, die beispielsweise durch eine Bewegung des Reaktionsgefäßes, durch eine Bewegung der in dem Reaktionsgefäß angeordneten Trägerplatte oder durch eine Bewegung der Lichtquelle bewerkstelligt werden kann, kann die Fokusebene innerhalb des Reaktionsgefäßes ebenfalls geändert werden. Bei dieser Variante ist folglich keine Refokussierung eines optional einzusetzenden optischen Systems erforderlich. Dadurch kann die Gefahr optischer Dejustagen reduziert werden.

In einer weiteren Verfahrensvariante werden die erste und/oder die weitere Lichtstrahlung auf einen definierten und vorbestimmbaren Bereich in der jeweiligen Fokusebene innerhalb der ersten photopolymerisierbaren Flüssigkeit und/oder der weiteren photopolymerisierbaren Flüssigkeit gelenkt. Das heißt, es kann ein spezifisches Lichtmuster vorgegeben werden, das auf die photopolymerisierbare Flüssigkeit trifft und an diesen Stellen zu einer Polymerisierung der Flüssigkeit zur Bildung eines Polymers bzw. eines Gels (der Matrix) dient. Ein derartiges Lichtmuster kann beispielsweise durch den Einsatz von Masken oder Blenden, aber auch durch den Einsatz einer gepulsten Lichtstrahlung oder die digitale Modulierung eines Lichtsignals erzeugt werden. An den Bereichen der photopolymerisierbaren Flüssigkeit, die von der Lichtstrahlung getroffen werden, kommt es zu einer Polymerisierung. An den anderen, nicht von der Lichtstrahlung getroffenen Bereichen verbleibt die photopolymerisierbare Flüssigkeit indes in ihrem nicht polymerisierten Zustand. Dadurch definiert die Lichtstrahlung die Bereiche, an denen ein Drucken der polymerisierten Struktur erfolgt. Mit einem derartigen lichtunterstützten Drucken sind weitaus höhere Auflösungen möglich, als dies bei den aus dem Stand der Technik bekannten Verfahren der Fall ist. Die Auflösung hängt dabei von der Wellenlänge des eingesetzten Lichtes ab. Sie ist selbst bei regelmäßig eingesetzten großen Wellenlängen besser als die Auflösung, die mit den herkömmlichen, aus dem Stand der Technik bekannten Verfahren, realisierbar ist. Je genauer die Lichtquelle fokussiert werden kann, desto größer ist die resultierende Auflösung. Beispielsweise können mit einem Laser sehr hohe Auflösungen erreicht werden.

Die Lichtstrahlung kann bei Bedarf über Spiegel auf die jeweilige Fokusebene gelenkt werden.

Das jeweils gewählte Belichtungsmuster kann beispielsweise von einem Computerprogramm bereitgestellt werden. So ist es denkbar, dass ein Anwender das herzustellende dreidimensionale Objekt mittels eines CAD-Programms erstellt. Das derart erstellte digitale Objekt wird dann von einem geeigneten Computerprogramm in einzelne Belichtungsebenen zerschnitten. Ferner wird jeder Ebene bzw. unterschiedlichen Orten einer jeden Ebene eine bestimmte photopolymerisierbare Flüssigkeit bzw. ein bestimmter Zelltyp zugeordnet. Zu diesen Informationen werden Steuerinformationen für einen Drucker erstellt, mittels dessen das beschriebene Verfahren durchgeführt wird. Diese Steuerinformationen geben vor, wann welche photopolymerisierbare Flüssigkeit in das Reaktionsgefäß eingebracht werden muss. Ferner geben diese Steuerinformationen vor, wann welches Bild einer Belichtungsebene auf die jeweilige Fokusebene im Reaktionsgefäß projiziert werden soll. Auf diese Weise lässt sich dann das zuvor am Computer erstellte digitale Objekt in ein reales dreidimensionales mehrzelliges Objekt überführen.

Erfindungsgemäß wird mehr als eine polymerisierte Struktur in derselben Schicht (das heißt, in derselben Fokusebene) erzeugt. Dazu erfolgen zunächst eine Polymerisierung einer ersten photopolymerisierbaren Flüssigkeit und beispielsweise ein Einbetten eines ersten Zelltyps in dem aus der ersten Flüssigkeit gebildeten Polymer. Anschließend wird die erste photopolymerisierbare Flüssigkeit aus dem Reaktionsgefäß entfernt und eine zweite photopolymerisierbare Flüssigkeit in das Reaktionsgefäß eingebracht. Nun werden nur solche Bereiche innerhalb der Fokusebene im Reaktionsgefäß belichtet, die zuvor nicht belichtet wurden und an denen folglich noch keine polymerisierte Struktur vorliegt. Dadurch können unterschiedliche Zelltypen bzw. unterschiedliche Matrices in ein und derselben Schicht erzeugt werden. Es werden folglich mehrere polymerisierte Strukturen in ein und derselben Schicht gebildet, so dass eine heterogene Schicht resultiert. Anschließend kann die zweite photopolymerisierbare Flüssigkeit aus dem Reaktionsgefäß entfernt und eine weitere photopolymerisierbare Flüssigkeit in das Reaktionsgefäß eingebracht werden. Der Füllstand dieser weiteren photopolymerisierbaren Flüssigkeit kann nun auf ein solches Niveau gebracht werden, dass die zuvor gebildete Schicht vollständig überdeckt ist. Dann kann die Fokusebene verschoben werden und eine weitere Schicht des zu erzeugenden dreidimensionalen mehrzelligen Objektes durch eine entsprechende polymerisierte Struktur aufgebaut werden. Dabei ist es grundsätzlich möglich, dass einzelne Schichten des erzeugten dreidimensionalen Objektes homogen (umfassend eine polymerisierte Struktur eines einzigen Typs) und andere Schichten heterogen (umfassend polymerisierte Strukturen unterschiedlichen Typs) bestehen, wobei die Anzahl der einzelnen Strukturen pro Schicht nicht begrenzt ist. In der Praxis haben sich neben einer einzigen polymerisierten Struktur pro Schicht heterogen zusammengesetzte Schichten mit 2, 3, 4, 5, 6, 7, 8, 9 oder 10 polymerisierten Strukturen als sinnvoll herausgestellt.

In einer Variante wird zumindest die erste Struktur der ersten Schicht, insbesondere aber jede Struktur der ersten Schicht, aus zwei unterschiedlichen Richtungen mit der ersten Lichtstrahlung bestrahlt. Dabei sind diese beiden unterschiedlichen Richtungen vorzugsweise einander entgegengesetzt. Mit einer derartigen Bestrahlung aus zwei unterschiedlichen Richtungen wird eine besonders feste Verankerung der ersten Schicht auf der inneren Oberfläche des Reaktionsgefäßes bzw. auf einer Trägerplatte, die in dem Reaktionsgefäß angeordnet ist, erreicht. Dadurch wird ein späterer sicherer Halt des gesamten erzeugten dreidimensionalen mehrzelligen Objektes an dem Reaktionsgefäß bzw. an einer Trägerplatte in dem Reaktionsgefäß erreicht, wodurch nachfolgende Untersuchungen an dem Objekt erleichtert werden. Typischerweise erfolgt die Bestrahlung bei einem nach oben offen en Reaktionsgefäß von oben. Die erste Schicht wird in dieser Variante dann vorzugsweise zusätzlich von unten durch den Boden des Reaktionsgefäßes hindurch bestrahlt. Dazu muss das Reaktionsgefäß aus einem Material gefertigt sein, das für die Lichtstrahlung mit der gewählten Wellenlänge durchlässig ist. Die nachfolgenden Schichten, die oberhalb der ersten Schicht angeordnet sind, werden dann vorzugsweise wiederum nur aus einer Richtung (nämlich vorzugsweise von oben) belichtet, damit die bereits gebildeten polymerisierten Strukturen nicht zwischen der Fokusebene der Lichtstrahlung und einer zur Aussendung der Lichtstrahlung eingesetzten Lichtquelle liegen und somit nicht von der Lichtstrahlung vor deren Fokusebene erneut durchstrahlt werden.

In einer Variante weisen die erste Lichtstrahlung und/oder die weitere Lichtstrahlung eine Wellenlänge im Bereich von 200 nm bis 1000 nm (also eine Wellenlänge, die zwischen dem UV-Bereich und dem Infrarot-Bereich liegt) auf. Mit derartigen Wellenlängen lassen sich die bevorzugt als Radikalbildner eingesetzten Substanzen besonders gut anregen, so dass Radikale gebildet werden, um eine Polymerisierung von Acrylfunktionen tragenden Ausgangssubstanzen zu ermöglichen.

Weitere geeignete Wellenlängen der eingesetzten Lichtstrahlung liegen im Bereich von 250 bis 950 nm, insbesondere von 300 bis 850 nm, insbesondere von 350 bis 800 nm, insbesondere von 400 bis 750 nm, insbesondere von 450 bis 700 nm, insbesondere von 500 bis 650 nm und ganz besonders von 500 bis 600 nm.

Da UV-Licht biologische Zellen schädigen kann, wird in einer Variante lediglich Licht mit einer Wellenlänge im sichtbaren Bereich, also von etwa 380 nm bis etwa 780 nm, eingesetzt. Dabei können auch UV-Filter vorgesehen sein, die UV-Anteile aus einer Lichtstrahlung herausfiltern, um möglicherweise schädigende UV-Strahlung sicher aus der eingesetzten Lichtstrahlung herauszufiltern.

Die zur Polymerisation eingesetzten Lichtstrahlungen können dieselbe Wellenlänge oder aber verschiedene Wellenlängen aus dem vorgenannten Wellenlängenbereich umfassen, um eine geeignete Polymerisation der unterschiedlichen photopolymerisierbaren Flüssigkeiten zu ermöglichen. Dabei können die einzelnen Lichtstrahlungen durch unterschiedliche oder aber durch ein und dieselbe Lichtquelle erzeugt werden. Es ist auch möglich, innerhalb einer Schicht (und damit innerhalb einer Fokusebene) nacheinander verschiedene Wellenlängen einzusetzen, um unterschiedliche photopolymerisierbare Flüssigkeiten in derselben Schicht zu polymerisieren, wenn eine heterogene Schicht aus unterschiedlichen polymerisierten Strukturen gebildet werden soll.

In einer Variante wird das Verfahren derart durchgeführt, dass während der Erzeugung des dreidimensionalen mehrzelligen Objektes mindestens ein funktionelles Element in das dreidimensionale mehrzellige Objekt eingebracht wird. Das funktionelle Element ist dabei ausgewählt aus der Gruppe bestehend aus Membranen, Kanälen, Poren, Sensoren, elektrisch leitenden Trägern und chemotaktischen Präparaten. Kanäle und Poren können beispielsweise in das Objekt integriert werden, indem bestimmte Bereiche der gebildeten polymerisierten Struktur in mehreren Schichten übereinander freigelassen werden.

Membranen können durch das Einbringen von Lipidmolekülen in die photopolymerisierbare Flüssigkeit gebildet werden.

Darüber hinaus können mit Hilfe der Photopolymerisation ebenfalls Salzbrücken innerhalb des Objekts eingebracht werden. Dies ist besonders einfach möglich, wenn die photopolymerisierbare Flüssigkeit Salze enthält, also salzhaltig ist. Auf diesem Wege kann nachfolgend eine elektrische Ableitung und Enervierung des gedruckten Objekts erfolgen.

Über bereits während des Herstellungsprozesses in das Objekt eingebrachte Sensoren braucht das hergestellte dreidimensionale Objekt nachträglich nicht mehr manipuliert zu werden, sondern kann unmittelbar über die bereits eingebrachten Sensoren ausgelesen werden. Dies erleichtert nachfolgende Analysen des dreidimensionalen Objektes erheblich.

Durch das Einbringen elektrisch leitender Träger, wie etwa Elektroden, wird es besonders einfach, bei einer nachfolgenden Untersuchung des gebildeten dreidimensionalen mehrzelligen Objektes das elektrische Potential bzw. die elektrischen Eigenschaften des Objektes zu analysieren.

Durch das Einbringen chemotaktischer Präparate, die in einer Variante in unterschiedlichen Schichten in unterschiedlichen Konzentrationen eingebracht werden können, um so einen Gradienten zu erzeugen, lässt sich das gezielte Wachstum von Zellen innerhalb des mehrzelligen dreidimensionalen Objektes nach dessen Fertigstellung ermöglichen. Wenn es sich bei dem chemotaktischen Präparat um einen Lockstoff handelt, übt er eine positive Chemotaxis aus, so dass sich die Zellen des dreidimensionalen Objektes zu Bereichen höherer Konzentration des Lockstoffes hin orientieren werden. Handelt es sich bei dem chemotaktischen Präparat indes um einen Schreckstoff, übt er eine negative Chemotaxis aus, so dass sich die Zellen in dem dreidimensionalen Objekt zu Bereichen niedrigerer Konzentration des Schreckstoffes bzw. zu Bereichen, in denen der Schreckstoff gar nicht vorhanden ist, orientieren werden. Dadurch lässt sich ein zielgerichtetes Wachstum von Zellen innerhalb des mehrzelligen Objektes erreichen.

Vorzugsweise wird zumindest ein Füllstandssensor eingesetzt, um die Flüssigkeitshöhe im Reaktionsgefäß stets genau zu erkennen. Aufgrund dieser Füllstandsinformationen kann dann die Fokusebene bestimmt werden, in der der nächste Polymerisationsschritt durchgeführt werden soll. Die von einem derartigen Füllstandssensor bereitgestellten Daten können auch dazu dienen, die Fokusebene automatisiert anzupassen. Über die von einem Füllstandssensor bereitgestellten Daten kann auch eine Pumpe gesteuert werden, die den Zufluss der photopolymerisierbaren Flüssigkeiten in das Reaktionsgefäß vermittelt. Auf diese Weise kann stets genau eine solche Menge der photopolymerisierbaren Flüssigkeiten in das Reaktionsgefäß eingebracht werden, die zum Aufbau der gerade gewünschten Schicht erforderlich ist. Dadurch werden Abfallmengen gering gehalten. Ferner wird dadurch eine kostengünstige Realisierung des gesamten Verfahrens ermöglicht.

Wie sich aus der vorherigen Darstellung des vorliegend beschriebenen Verfahrens ergibt, kann dieses Verfahren vollständig automatisiert durchgeführt werden, so dass ein Eingriff eines Benutzers nicht erforderlich ist. Dies erleichtert die Verfahrensanwendung zusätzlich.

Die Zeitdauer, während der die Lichtstrahlung auf die jeweilige Fokusebene eingestrahlt wird, kann an die jeweiligen Anforderungen der eingesetzten photopolymerisierbaren Flüssigkeiten angepasst werden. Das heißt, jedem Material wird zur Aushärtung eine solche Zeit zugestanden, die für die gewünschte Polymerisierung erforderlich und sinnvoll ist.

Wenn ein Träger innerhalb des Reaktionsgefäßes angeordnet ist, kann sich beim Anheben dieses Trägers relativ zum Reaktionsgefäß ein Unterdruck zwischen einem umgebenden Flüssigkeitsbett und den bereits polymerisierten Strukturen auf dem Träger ergeben. Durch das Absaugen der Reste der für den vorherigen Polymerisationsschritt noch im Reaktionsgefäß befindlichen photopolymerisierbaren Flüssigkeit und das Einbringen einer neuen photopolymerisierbaren Flüssigkeit kann ein potentiell herrschender Unterdruck jedoch entspannt werden. Dadurch kann der Träger relativ zum Reaktionsgefäß bewegt werden, ohne dass ein Abreißen der bereits polymerisierten Strukturen des dreidimensionalen Objektes von dem Träger befürchtet werden muss.

Wenn das dreidimensionale Objekt auf einer Trägerplatte erzeugt wird, kann diese Trägerplatte nach Ende des Herstellungsprozesses komplett aus der noch verbliebenen Flüssigkeit im Reaktionsgefäß herausgehoben werden. Anschließend kann das erzeugte Objekt vom Anwender von der Trägerplatte entfernt werden. Damit das Objekt bei der Abnahme von einer Trägerplatte nicht zerstört wird, kann die Trägerplatte derart ausgestaltet sein, dass ein steriler Luftstrom zwischen der Oberfläche der Trägerplatte und der Unterseite des erzeugten dreidimensionalen Objektes durchgeführt werden kann. Dadurch wird das Objekt dann gleichmäßig von der Trägerplatte weggedrückt, wodurch ein schonendes Entfernen des dreidimensionalen Objektes von der Trägerplatte gewährleistet ist.

Die der Erfindung zugrunde liegende Aufgabe wird auch durch eine Vorrichtung zur Erzeugung eines dreidimensionalen mehrzelligen Objektes aus photopolymerisierbaren Flüssigkeiten mit den nachfolgenden Merkmalen gelöst.

Eine derartige Vorrichtung weist ein Reaktionsgefäß und eine Lichtquelle auf, die derart angeordnet ist, dass sie im Betrieb der Vorrichtung Licht in das Reaktionsgefäß einstrahlen kann, wobei dieses Licht auf eine Fokusebene innerhalb des Reaktionsgefäßes fokussiert wird. Die Vorrichtung weist ferner ein Reservoir für unterschiedliche photopolymerisierbare Flüssigkeiten auf. Ferner ist eine Pumpe vorgesehen, die sowohl mit dem Reservoir als auch mit dem Reaktionsgefäß in Strömungsverbindung gebracht werden kann. Hierzu können geeignete Ventile zwischen der Pumpe und dem Reservoir bzw. zwischen der Pumpe und dem Reaktionsgefäß vorgesehen sein. Dadurch ist es möglich, mittels der Pumpe die unterschiedlichen photopolymerisierbaren Flüssigkeiten in das Reaktionsgefäß einzubringen und aus dem Reaktionsgefäß auszutragen. Schließlich ist noch eine Steuereinheit zur Steuerung der Lichtquelle und der Pumpe vorgesehen.

Die Steuerungseinheit ist dabei so konfiguriert ist, um
- die Lichtquelle in einem ersten Polymerisationsschritt zu steuern, um Licht auf eine erste Fokusebene, die innerhalb eines mit der ersten Flüssigkeit gefüllten Bereiches des Reaktionsgefäßes liegt, zu strahlen, um eine erste polymerisierbare Struktur zu erzeugen;
- eine weitere photopolymerisierbare Flüssigkeit in das Reaktionsgefäß einzubringen,
- die Lichtquelle in einem weiteren, zweiten Polymerisationsschritt so zu steuern, dass Licht auf Bereiche innerhalb einer Fokusebene strahlt, auf die in dem ersten Polymerisationsschritt in derselben Fokusebene kein Licht gestrahlt wurde, um eine weitere polymerisierte Struktur in einer weiteren Schicht zu erzeugen,wobei die weitere polymerisierte Struktur unmittelbar auf der zuvor erzeugten ersten polymerisierten Struktur angeordnet und mit dieser verbunden ist, und wobei mehr als eine polymerisierte Struktur in derselben Schicht erzeugt wird

Die grundsätzliche Funktion der einzelnen Elemente dieser Vorrichtung wurde bereits im Zusammenhang mit den Erläuterungen zu dem oben beschriebenen Verfahren dargelegt.

Wenn das Reaktionsgefäß eine Kavität einer Mikrotiterplatte ist, können in einer Variante eine zahlreiche verschiedene Leitungen vorgesehen sein, die über unterschiedliche Ventile ansteuerbar sind, so dass ein gleichzeitiges Füllen bzw. Entleeren der unterschiedlichen Reaktionsgefäße möglich ist. Ferner kann auch eine einzige Einfüll- bzw. Absaugeinrichtung vorgesehen sein, die zu den unterschiedlichen Kavitäten einer Mikrotiterplatte bewegt werden kann.

Vorzugsweise erfolgen eine Zufuhr und/oder ein Entfernen von photopolymerisierbaren Flüssigkeiten aus dem Reaktionsgefäß in einem bodennahen Bereich des Reaktionsgefäßes. Denn auf diese Weise kann einerseits sichergestellt werden, dass auch Restmengen einer zu entfernenden photopolymerisierbaren Flüssigkeit aus dem Reaktionsgefäß entfernt werden. Darüber hinaus kann auf diese Weise eine schonende Zufuhr einer neuer photopolymerisierbaren Flüssigkeit in das Reaktionsgefäß gewährleistet werden, so dass bereits polymerisierte Strukturen durch die neu zugeführte Flüssigkeit nicht in Mitleidenschaft gezogen werden.

In einer Variante ist die Lichtquelle dafür vorgesehen und eingerichtet, Licht unterschiedlicher Wellenlänge zu emittieren. Dabei kann die Wellenlänge des zu emittierenden Lichtes durch einen Benutzer oder durch ein Steuerprogramm vorgegeben werden. Damit lassen sich unterschiedliche Polymerisationswellenlängen realisieren, ohne dass hierfür unterschiedliche Lichtquellen eingesetzt werden müssen.

In einer Variante ist mindestens ein Spiegel vorgesehen, um die erste und/oder die weitere Lichtstrahlung auf die photopolymerisierbaren Flüssigkeiten zu lenken. Auf diese Weise lassen sich noch mehr unterschiedliche Anordnungen von Lichtquelle und Reaktionsgefäß realisieren.

Die zuvor beschriebene Vorrichtung eignet sich insbesondere zur Erzeugung eines künstlichen Organs oder eines Schwangerschaftsmodells, wie dies bereits oben in Zusammenschau mit dem dort beschriebenen Verfahren erläutert wurde. Dabei kann das künstliche Organ ein gesundes Organmodell oder ein Krankheitsmodell sein.

Bevorzugte oder alternative Ausgestaltungen des hier beschriebenen Verfahrens sind in analoger Weise auf die beschriebene Vorrichtung bzw. die beschriebene Verwendung übertragbar, und umgekehrt. Dabei sind beliebige Kombinationen der einzelnen Varianten vorgesehen.

Weitere Einzelheiten der vorliegenden Erfindung sollen anhand von Ausführungsbeispielen und entsprechender Figuren näher erläutert werden. Es zeigen:
- Figur 1: ein erstes Ausführungsbeispiel einer Vorrichtung zur Erzeugung eines dreidimensionalen mehrzelligen Objektes aus photopolymerisierbaren Flüssigkeiten,
- Figur 2: ein zweites Ausführungsbeispiel einer Vorrichtung zur Erzeugung eines dreidimensionalen mehrzelligen Objektes aus photopolymerisierbaren Flüssigkeiten und
- Figur 3: ein Ausführungsbeispiel einer Verfahrensvariante, bei der eine Trägerstruktur gedruckt wird.

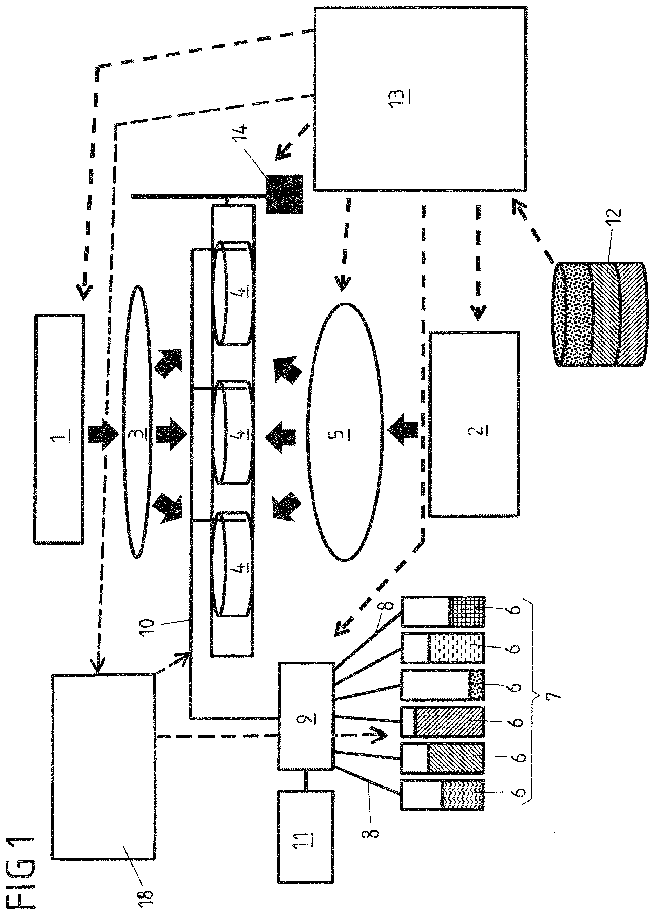

Die Figur 1 zeigt den schematischen Aufbau eines 3D-Druckers als Vorrichtung zur Erzeugung eines dreidimensionalen mehrzelligen Objektes aus photopolymerisierbaren Flüssigkeiten. Der 3D-Drucker weist eine erste Lichtquelle 1 und eine zweite Lichtquelle 2 auf. Licht, welches von der ersten Lichtquelle 1 emittiert wird, wird über eine erste Linse 3 auf eine Vielzahl von Reaktionsgefäßen 4 geleitet, von denen in der Darstellung der Figur 1 lediglich drei gezeigt sind. In gleicher Weise wird Licht, welches von der zweiten Lichtquelle 2 emittiert wird, über eine zweite Linse 5 auf die Reaktionsgefäße 4 geleitet. Statt zweier verschiedener Lichtquellen 1, 2 könnte auch eine einzige Lichtquelle eingesetzt werden, wobei der Strahlengang dann derart konstruiert wäre, dass das von dieser einzigen Lichtquelle emittierte Licht wahlweise von der Oberseite der Reaktionsgefäße 4 und/oder von der Unterseite der Reaktionsgefäße 4 auf die Reaktionsgefäße 4 geleitet würde.

Die erste Lichtquelle 1 und die zweite Lichtquelle 2 können Licht unterschiedlicher Wellenlänge emittieren, wobei die Wellenlänge automatisch reguliert werden kann.

Eine Vielzahl von unterschiedlichen Kammern 6, die jeweils unterschiedliche Ausgangsflüssigkeiten als photopolymerisierbare Flüssigkeiten enthalten, bildet zusammen ein Reservoir 7, das über eine der Anzahl der Kammern 6 entsprechende Anzahl von Leitungen 8 mit einer Pumpe 9 verbunden ist. Mittels der Pumpe 9 können über die Leitungen 8 die in den Kammern 6 des Reservoirs 7 enthaltenen photopolymerisierbaren Flüssigkeiten zu den Reaktionsgefäßen 4 transportiert werden. Hierfür sind die Reaktionsgefäße 4 mit der Pumpe 9 über ein entsprechendes Leitungssystem 10 verbunden. Die Pumpe 9 ist ferner mit einem Abfallbehälter 11 verbunden, in den nicht mehr benötigte Flüssigkeitsreste eingebracht werden können. Denn die Pumpe 9 dient auch dazu, nicht mehr benötigte photopolymerisierbare Flüssigkeit aus den Reaktionsgefäßen 4 über das Leitungssystem 10 herauszusaugen und dann dem Abfallbehälter 11 zuzuführen.

Im Betrieb des in der Figur 1 gezeigten 3D-Druckers werden zunächst Daten über ein mittels eines CAD-Programms erzeugtes digitales Objekt 12 an eine zentrale Steuereinheit 13 übermittelt. In dieser zentralen Steuereinheit 13 kann dann eine Zerlegung des digitalen Objektes 12 in einzelne Ebenen erfolgen, sofern dies durch die übermittelten Informationen noch nicht geschehen ist. Die zentrale Steuereinheit 13 dient dann zur Ansteuerung der ersten Lichtquelle 1, der zweiten Lichtquelle 2 und der Pumpe 9. Darüber hinaus kann auch die zweite Linse 5 von der zentralen Steuereinheit 13 bewegt werden, falls dies von einem Anwender gewünscht wird. Nun wird die für die erste Schicht des zu erzeugenden dreidimensionalen Objektes benötigte photopolymerisierbare Flüssigkeit, welche die in die erste Schicht einzubringenden Zellen bereits enthält, aus der entsprechenden Kammer 6 des Reservoirs 7 mittels der Pumpe 9 gesaugt und über die entsprechende Leitung 8 und das Leitungssystem 10 zu den einzelnen Reaktionsgefäßen 4 befördert. Anschließend wird sowohl Licht von der ersten Lichtquelle 1 als auch Licht von der zweiten Lichtquelle 2 auf die Reaktionsgefäße 4 fokussiert, so dass eine Polymerisierung der in den Reaktionsgefäßen 4 befindlichen photopolymerisierbaren Flüssigkeit erfolgt und die dadurch gebildete polymerisierte Struktur fest an der Innenseite der Reaktionsgefäße 4 anheftet. Anschließend wird die noch verbliebene nicht polymerisierte Flüssigkeit durch die Pumpe 9 über das Leitungssystem aus den Reaktionsgefäßen 4 herausgesaugt und dem Abfallbehälter 11 zugeführt.

Nun wird entsprechend der von der zentralen Steuereinheit 13 vorgegebenen Daten eine weitere photopolymerisierbare Flüssigkeit aus der entsprechenden Kammer 6 des Reservoirs 7 durch die Pumpe 9 aufgesaugt und über die entsprechende Leitung 8 und das Leitungssystem 10 wiederum den Reaktionsgefäßen 4 zugeführt. Nun sorgt die zentrale Steuereinheit 13 dafür, dass die Fokusebene des auf die Reaktionsgefäße eingestrahlten Lichtes verändert wird. Zu diesem Zwecke steuert die zentrale Steuereinheit 13 einen Motor 14 an, der für ein Absenken der Reaktionsgefäße 4 um eine Höhe sorgt, die der Dicke der im ersten Schritt erzeugten polymerisierten Struktur der ersten Schicht entspricht. Nun erfolgt ein Einstrahlen von Licht aus der ersten Lichtquelle 1 von oben auf die Reaktionsgefäße 4, um eine zweite Schicht aus einer polymerisierten Struktur zu erzeugen, die unmittelbar oberhalb der ersten Schicht gebildet ist und mit der ersten Schicht mittels einer chemische Reaktionen kovalent verbunden wird.

Die zweite Lichtquelle 2 wird für diesen Schritt nicht mehr benötigt, da eine zweiseitige Bestrahlung nur für die unterste Schicht durchgeführt werden soll, da diese Schicht besonders fest an der Innenseite der Reaktionsgefäße 4 haften soll. Nachfolgend wird noch nicht polymerisierte Flüssigkeit wieder aus den Reaktionsgefäßen 4 herausgepumpt und entsprechend den Vorgaben der zentralen Steuereinheit 13 eine weitere polymerisierbare Flüssigkeit in die Reaktionsgefäße 4 eingebracht. Dann werden die Reaktionsgefäße 4 wieder abgesenkt, so dass sich die Fokusebene verändert und eine weitere Schicht gebildet werden kann. Diese Schritte werden so lange wiederholt, bis das gewünschte dreidimensionale Objekt erzeugt ist.

Wie oben erläutert, können dabei auch mehrere Polymerisationsschritte in derselben Schicht nacheinander durchgeführt werden, um eine heterogene Schicht aus unterschiedlichen polymerisierten Strukturen zu erzeugen. Ferner können verschiedene, aufeinander folgende Schichten aus derselben photopolymerisierbaren Flüssigkeit erzeugt werden. In einem solchen Fall ist es nicht nötig, die in einem ersten Polymerisationsvorgang noch nicht polymerisierte Flüssigkeit aus den Reaktionsgefäßen 4 abzusaugen. Vielmehr können die Reaktionsgefäße 4 einfach abgesenkt werden, um die Fokusebene zu ändern, so dass dann unter Verwendung der Reste der noch in den Reaktionsgefäßen befindlichen photopolymerisierbaren Flüssigkeit eine weitere Schicht einer polymerisierten Struktur auf der zuvor gebildeten Schicht erzeugt wird.

Die zentrale Steuereinheit 13 dient darüber hinaus auch der Ansteuerung einer Temperiereinheit 18, die die Reaktionsgefäße 4 bzw. einen die Reaktionsgefäße 4 umgebenden Raum und/oder das Reservoir 7 und/oder die Kammern 6 des Reservoirs 7 kühlen und/oder heizen kann, um so für definierte Reaktionsbedingungen zu sorgen. Die Temperiereinheit 18 ermöglicht auf besonders einfache Weise den Einsatz temperaturabhängiger Gelbildner und die Bildung temperaturabhängiger, metastabiler Gele.

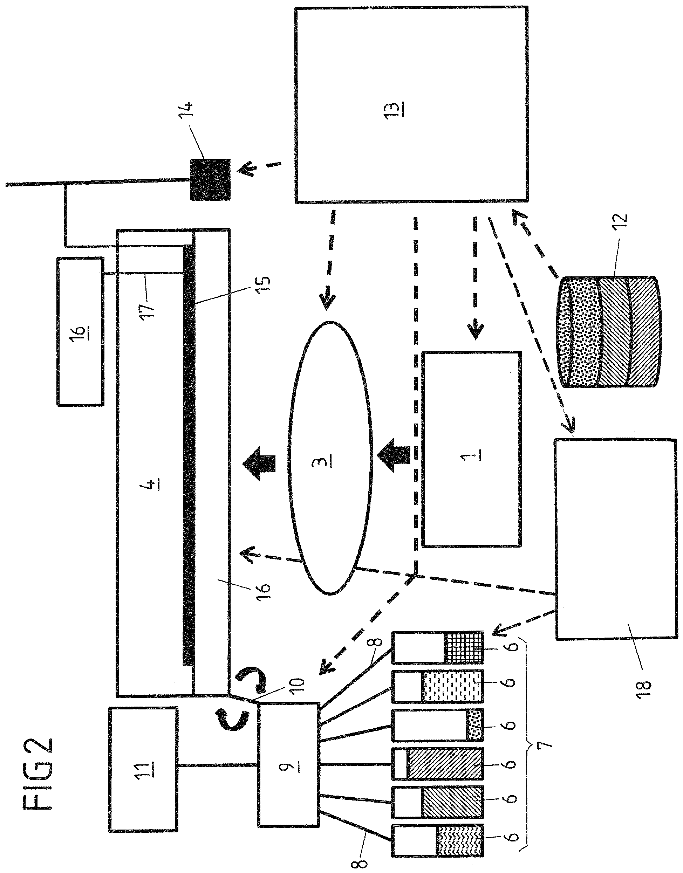

Die Figur 2 zeigt einen weiteren 3D-Drucker als weiteres Ausführungsbeispiel einer Vorrichtung zur Erzeugung eines dreidimensionalen mehrzelligen Objektes aus photopolymerisierbaren Flüssigkeiten. Dabei werden gleiche Elemente mit den gleichen Bezugszeichen wie in der Figur 1 versehen, wobei diesbezüglich auf die obigen Erläuterungen in Zusammenschau mit der Figur 1 verwiesen wird.

Der in der Figur 2 dargestellte 3D-Drucker unterscheidet sich von dem in der Figur 1 dargestellten 3D-Drucker insbesondere in der Ausbildung des Reaktionsgefäßes 4. Denn bei dem in der Figur 2 dargestellten 3D-Drucker ist eine Trägerplatte 15 innerhalb des Reaktionsgefäßes 4 angeordnet, die als Unterlage für das zu erzeugende dreidimensionale Objekt dient. Dabei erfolgt eine Bestrahlung mit Licht aus der ersten Lichtquelle 1 von einer Unterseite des Reaktionsgefäßes 4. Das heißt, das zu erzeugende dreidimensionale Objekt wird mit der Oberseite nach unten innerhalb des Reaktionsgefäßes 4 erzeugt. Zunächst wird also die unterste Schicht des zu erzeugenden Objektes auf der Trägerplatte 15 polymerisiert. Anschließend wird die Trägerplatte 15 mittels des Motors 14 angehoben, so dass dann die nächste Schicht auf der bereits an der Trägerplatte 15 anheftenden Schicht erzeugt wird. Das heißt, hier erfolgt eine Verschiebung der Fokusebene des durch die Lichtquelle 1 in das Reaktionsgefäß 4 eingestrahlten Lichtes durch ein Anheben der Trägerplatte 15. Dabei wird die Trägerplatte 15 nur so weit angehoben, dass eine auf ihr bereits ausgebildete Schicht aus einer polymerisierten Struktur gerade die Oberfläche einer sich im Reaktionsgefäß 4 befindlichen polymerisierbaren Flüssigkeit 16 berührt. Wenn nun das Licht aus der Lichtquelle 1 in das Reaktionsgefäß 4 eingestrahlt wird, wird die dadurch erzeugte weitere Schicht aus einer polymerisierten Struktur unmittelbar auf der bereits zuvor erzeugten Schicht abgeschieden, so dass eine kovalente Verbindung beider Schichten miteinander erfolgen kann, wodurch das letztlich erzeugte Objekt eine hohe Stabilität aufweist.

Da bei dem 3D-Drucker der Figur 2 nur ein einzelnes Reaktionsgefäß 4 vorgesehen ist, handelt es sich bei dem Leitungssystem 10, welches die Pumpe 9 mit dem Reaktionsgefäß 4 verbindet, auch lediglich um eine einzelne Leitung.

Damit das auf der Trägerplatte 15 erzeugte Objekt gut von der Trägerplatte 15 entfernt werden kann, ist zudem eine sterile Luftdruckquelle 16 vorgesehen, die über eine Luftdruckleitung 17 mit der Trägerplatte 15 in Strömungsverbindung gebracht werden kann. Wenn das dreidimensionale Objekt fertig erzeugt ist, kann über die sterile Luftdruckquelle 16 Luft zwischen eine Unterseite der Trägerplatte 15 und die erste Schicht des erzeugten Objektes gedrückt werden, so dass das Objekt leicht von der Trägerplatte 15 entfernt werden kann.

Die zentrale Steuereinheit 13 wird sowohl beim Ausführungsbeispiel der Figur 1 als auch beim Ausführungsbeispiel der Figur 2 neben den bereits genannten Funktionen auch dafür eingesetzt, das von der Lichtquelle erzeugte Bild bzw. Muster, die Belichtungszeitdauer, die Höhe der Reaktionsgefäße 4 bzw. des Trägers 15 innerhalb des Reaktionsgefäßes 4, die Fokusebene, die Füllhöhe der photopolymerisierbaren Flüssigkeit 16 innerhalb des Reaktionsgefäßes 4, die Auswahl der photopolymerisierbaren Flüssigkeit und/oder die in den Leitungen 8 und dem Leitungssystem 10 vorgesehenen Ventile zu steuern. Auf diese Weise können die 3D-Drucker vollautomatisch arbeiten und aufgrund entsprechend zugeführter Informationsdaten ein dreidimensionales Objekt ohne Interaktion mit einem Benutzer erzeugen.

Wie auch beim Ausführungsbeispiel der Figur 1 ist eine Temperiereinheit 18 vorgesehen. Es wird diesbezüglich auf die obigen Erläuterungen verwiesen.

An dem nachfolgenden erläuterten weiteren Ausführungsbeispiel wird eine mögliche Temperatursensitivität der eingesetzten photopolymersierbaren Flüssigkeit dargestellt.

Durch Zusatz einer temperatursensitiven, insbesondere einer invers-temperatursensitiven, Substanz kann die Erzeugung von hängenden Objekten und Hohlkammern zusätzlich verbessert werden. Hierbei kann beispielsweise eine Substanz wie etwa ein Poloxamer in einer solchen Konzentration zugemischt werden, dass die photopolymerisierbare Flüssigkeit oder eine nicht photopolymerisierbare Flüssigkeit auch ohne Lichteinstrahlung in einem gewünschten Temperaturbereich geliert.

Beispielhaft kann das Verfahren so ablaufen: Wenn eine Gelierung bei einer Temperatur von ca. 20 °C erreicht werden soll, mischt man der photopolymerisierbaren Flüssigkeit ein Poloxamer in einer Konzentration bei, so dass die Flüssigkeit in diesem Bereich geliert. Es sind auch Mischungen aus mehreren Poloxameren möglich. Wenn möglich, kann die Flüssigkeit zunächst auf eine Temperatur unter den Gelierpunkt gekühlt werden. Ist eine hängende Struktur innerhalb des Objekts gewünscht, kann die Flüssigkeit, die den temperatursensitiven Gelbildner enthält, auf eine Temperatur erwärmt werden, die über der Geliertemperatur liegt. Die Flüssigkeit geliert dann. Parallel dazu kann die Flüssigkeit ebenfalls photopolymerisiert werden. Sollte ein Bereich der temperatursensitiven Flüssigkeit nicht photopolymerisiert werden, ist diese bei der erhöhten Temperatur zwar fest, bei einer Absenkung der Temperatur unterhalb der Geliertemperatur jederzeit wieder verflüssigbar. So kann der temperatursensitive, gelierte Teil als Stützstruktur bis zum Ende des Druckprozesses fungieren. Nach Abschluss des Druckens kann die Temperatur wieder unter die oben genannte beispielhafte Geliertemperatur von 20 °C gesenkt werden. Als Folge verflüssigt sich der nicht polymerisierte, temperatursensitive Teil der Flüssigkeit wieder und kann abgepumpt werden. Wird das Gel verflüssigt, wird die Stützstruktur entfernt und das ehemals gestützte Teil des gedruckten Objektes, das nun photopolymerisiert ist, hängt frei.

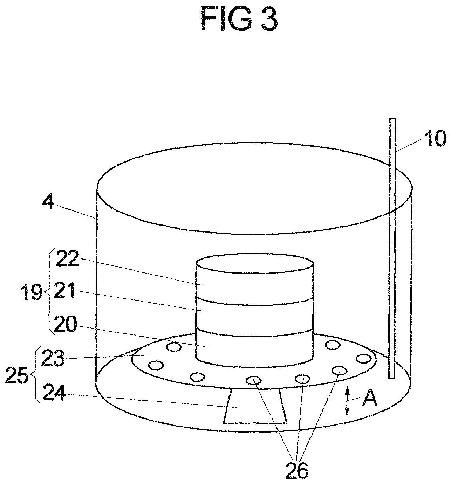

Die Figur 3 zeigt ein in einem Reaktionsgefäß 4 gedrucktes Objekt 19. Das gedruckte Objekt 19 besteht aus mehreren übereinanderliegenden polymerisierten Strukturen 20, 21, 22, die in der Darstellung der Figur 3 lediglich schematisch gezeigt sind. Die unterste polymerisierte Struktur ist auf einer Plattform 23 gebildet, die als Trägerplatte dient. Die Plattform 23 ist über einen mittig angeordneten Standfuß 24 mit dem Boden des Reaktionsgefäßes 4 verbunden. Durch den Standfuß 24 ist ein Abstand A zwischen der Plattform 23 und dem Boden des Reaktionsgefäßes 4 ausgebildet.

Die Plattform 23 und der Standfuß 24 bilden zusammen einen Träger 25, der auch als Trägerstruktur bezeichnet werden kann. In der Plattform 23 sind Löcher 26 ausgebildet, durch die hindurch Flüssigkeiten auf den Boden des Reaktionsgefäßes 4 fließen können. Die Flüssigkeiten können dann besonders einfach durch ein Leitungssystem 10 (vergleiche hierzu auch die Figuren 1 und 2) abgesaugt werden. Ferner können so frische Flüssigkeiten durch das Leitungssystem 10 einfach in das Reaktionsgefäß eingebracht werden und sich gut verteilen, da dann die unterste polymerisierte Struktur 20 eine Flüssigkeitsverteilung nicht behindert.

## Patentansprüche

1. Verfahren zur Erzeugung eines dreidimensionalen mehrzelligen Objektes, mit den folgenden Schritten:
a) Einbringen einer ersten photopolymerisierbaren Flüssigkeit in ein Reaktionsgefäß (4),
b) Fokussieren einer ersten Lichtstrahlung auf eine erste Fokusebene, die innerhalb eines mit der ersten Flüssigkeit gefüllten Bereiches des Reaktionsgefäßes (4) liegt,
c) Erzeugen einer ersten polymerisierten Struktur in einer ersten Schicht in dem Reaktionsgefäß (4) durch die erste Lichtstrahlung,
d) Einbringen einer weiteren photopolymerisierbaren Flüssigkeit in das Reaktionsgefäß (4), so dass eine zuvor erzeugte polymerisierte Struktur zumindest teilweise mit der weiteren photopolymerisierbaren Flüssigkeit bedeckt ist,
e) Fokussieren einer weiteren Lichtstrahlung auf eine weitere Fokusebene, die innerhalb eines mit der weiteren Flüssigkeit gefüllten Bereiches des Reaktionsgefäßes (4) liegt,
f) Erzeugen einer weiteren polymerisierten Struktur in einer weiteren Schicht in dem Reaktionsgefäß (4) durch die weitere Lichtstrahlung, wobei die weitere polymerisierte Struktur unmittelbar auf der zuvor erzeugten polymerisierten Struktur angeordnet und mit dieser verbunden ist,
g) Wiederholen der Schritte d) bis f) mit jeweils einer weiteren photopolymerisierbaren Flüssigkeit, bis das dreidimensionale mehrzellige Objekt erzeugt ist,
wobei die erste photopolymerisierbare Flüssigkeit und/oder zumindest eine der weiteren photopolymerisierbaren Flüssigkeiten biologische Zellen enthalten
**dadurch gekennzeichnet,**
**dass** mehr als eine polymerisierte Struktur in derselben Schicht erzeugt wird.

2. Verfahren nach Anspruch 1, **dadurch gekennzeichnet, dass** die erste photopolymerisierbare Flüssigkeit und zumindest eine der weiteren photopolymerisierbaren Flüssigkeiten unterschiedliche Flüssigkeiten sind.

3. Verfahren nach Anspruch 1 oder 2, **dadurch gekennzeichnet, dass** die weitere photopolymerisierbare Flüssigkeit erst dann in das Reaktionsgefäß (4) eingebracht wird, wenn die zuvor im Reaktionsgefäß (4) befindliche photopolymerisierbare Flüssigkeit aus dem Reaktionsgefäß (4) entfernt wurde.

4. Verfahren nach einem der vorhergehenden Ansprüche, **dadurch gekennzeichnet, dass** eine Trägerplatte (15) in dem Reaktionsgefäß (4) angeordnet ist, an die die erste polymerisierte Struktur gebunden wird.

5. Verfahren nach einem der vorhergehenden Ansprüche, **dadurch gekennzeichnet, dass** eine Refokussierung in einem optischen System (5), welches zwischen einer Lichtquelle (2) zur Erzeugung der ersten und/oder der weiteren Lichtstrahlung und dem Reaktionsgefäß (4) angeordnet ist, durchgeführt wird, um die Fokusebene innerhalb des Reaktionsgefäßes (4) zu ändern.

6. Verfahren nach einem der vorhergehenden Ansprüche, **dadurch gekennzeichnet, dass** eine Relativbewegung zwischen dem Reaktionsgefäß (4) oder einer in dem Reaktionsgefäß (4) angeordneten Trägerplatte (15) einerseits und einer Lichtquelle (1, 2) zur Erzeugung der ersten und/oder der weiteren Lichtstrahlung andererseits durchgeführt wird, um die Fokusebene zu ändern.

7. Verfahren nach einem der vorhergehenden Ansprüche, **dadurch gekennzeichnet, dass** die erste und/oder die weitere Lichtstrahlung auf einen definierten und vorbestimmbaren Bereich in der jeweiligen Fokusebene innerhalb der ersten photopolymerisierbaren Flüssigkeit und/oder der weiteren photopolymerisierbaren Flüssigkeit gelenkt wird.

8. Verfahren nach einem der vorhergehenden Ansprüche, **dadurch gekennzeichnet, dass** zumindest die erste polymerisierte Struktur in der ersten Schicht aus zwei Richtungen mit der ersten Lichtstrahlung bestrahlt wird.

9. Verfahren nach einem der vorhergehenden Ansprüche, **dadurch gekennzeichnet, dass** die erste Lichtstrahlung und/oder die weitere Lichtstrahlung eine Wellenlänge im Bereich von 200 bis 1000 nm aufweist.

10. Verfahren nach einem der vorhergehenden Ansprüche, **dadurch gekennzeichnet, dass** die erste Lichtstrahlung und/oder die weitere Lichtstrahlung eine Wellenlänge im Bereich von 380 bis 780 nm aufweist.

11. Verfahren nach einem der vorhergehenden Ansprüche, **dadurch gekennzeichnet, dass** während der der Erzeugung des dreidimensionalen mehrzelligen Objektes mindestens ein Element in das dreidimensionale mehrzellige Objekt integriert wird, das ausgewählt ist aus der Gruppe bestehend aus Membranen, Kanälen, Poren, Sensoren, elektrisch leitenden Trägern und chemotaktischen Präparaten.

12. Vorrichtung zur Erzeugung eines dreidimensionalen mehrzelligen Objektes aus photopolymerisierbaren Flüssigkeiten, mit
• einem Reaktionsgefäß (4),
• einer Lichtquelle (1, 2), die derart angeordnet ist, dass sie im Betrieb der Vorrichtung Licht in einer Fokusebene in das Reaktionsgefäß (4) einstrahlen kann,
• einem Reservoir (7) für unterschiedliche photopolymerisierbare Flüssigkeiten,
• einer Pumpe (9), die mit dem Reservoir (7) und dem Reaktionsgefäß (4) in Strömungsverbindung gebracht werden kann, um die unterschiedlichen photopolymerisierbaren Flüssigkeiten in das Reaktionsgefäß (4) einzubringen und aus dem Reaktionsgefäß auszutragen, und
• einer Steuereinheit (13) zur Steuerung der Lichtquelle (1, 2) und der Pumpe (9), **dadurch gekennzeichnet,**
**dass** die Steuerungseinheit konfiguriert ist, um
- die Lichtquelle in einem ersten Polymerisationsschritt zu steuern, um Licht auf eine erste Fokusebene, die innerhalb eines mit der ersten Flüssigkeit gefüllten Bereiches des Reaktionsgefäßes (4) liegt, zu strahlen, um eine erste polymerisierbare Struktur zu erzeugen;
- durch Ansteuerung der Pumpe eine weitere photopolymerisierbare Flüssigkeit in das Reaktionsgefäß einzubringen,
- die Lichtquelle in einem weiteren, zweiten Polymerisationsschritt so zu steuern, dass Licht auf Bereiche innerhalb einer Fokusebene strahlt, auf die in dem ersten Polymerisationsschritt in derselben Fokusebene kein Licht gestrahlt wurde, um eine weitere polymerisierte Struktur in einer weiteren Schicht zu erzeugen,wobei die weitere polymerisierte Struktur unmittelbar auf der zuvor erzeugten ersten polymerisierten Struktur angeordnet und mit dieser verbunden ist, und wobei mehr als eine polymerisierte Struktur in derselben Schicht erzeugt wird.

13. Vorrichtung nach Anspruch 12, **dadurch gekennzeichnet, dass** die Lichtquelle (1, 2) dafür vorgesehen und eingerichtet ist, Licht unterschiedlicher Wellenlänge zu emittieren, wobei die Wellenlänge des zu emittierenden Lichts vorgegeben werden kann.

14. Verwendung einer Vorrichtung gemäß Anspruch 12 oder 13 zur Erzeugung eines künstlichen Organs oder eines Schwangerschaftsmodells.

## Claims

1. Method for creating a three-dimensional multi-cell object, comprising the following steps:
a) introducing a first photopolymerizable liquid into a reaction vessel (4),
b) focusing a first light radiation onto a first focal plane located within a region of the reaction vessel (4) filled with the first liquid,
c) creating a first polymerized structure in a first layer in the reaction vessel (4) by means of the first light radiation,
d) introducing a further photopolymerizable liquid into the reaction vessel (4) such that a previously created polymerized structure is at least partly covered by the further photopolymerizable liquid,
e) focusing a further light radiation onto a further focal plane located within a region of the reaction vessel (4) filled with the further liquid,
f) creating a further polymerized structure in a further layer in the reaction vessel (4) by means of the further light radiation, wherein the further polymerized structure is disposed directly on the previously created polymerized structure and connected to the latter,
g) repeating steps d) to f) with a further photopolymerizable liquid in each case until the three-dimensional multi-cell object is created, wherein the first photopolymerizable liquid and/or at least one of the further photopolymerizable liquids contain biological cells,
**characterized in that**
more than one polymerized structure is created in the same layer.

2. Method according to Claim 1, **characterized in that** the first photopolymerizable liquid and at least one of the further photopolymerizable liquids are different liquids.

3. Method according to Claim 1 or 2, **characterized in that** the further photopolymerizable liquid is only introduced into the reaction vessel (4) once the polymerizable liquid previously situated in the reaction vessel (4) has been removed from the reaction vessel (4).

4. Method according to any one of the preceding claims, **characterized in that** a carrier plate (15) is disposed in the reaction vessel (4), the first polymerized structure being bound thereto.

5. Method according to any one of the preceding claims, **characterized in that**, for the purposes of changing the focal plane within the reaction vessel (4), refocusing is performed in an optical system (5) disposed between a light source (2) for producing the first and/or the further light radiation and the reaction vessel (4).

6. Method according to any one of the preceding claims, **characterized in that**, for the purposes of changing the focal plane, a relative motion is performed between, firstly, the reaction vessel (4) or a carrier plate (15) disposed in the reaction vessel (4) and, secondly, a light source (1, 2) for producing the first and/or the further light radiation.

7. Method according to any one of the preceding claims, **characterized in that** the first and/or the further light radiation is steered onto a defined and predeterminable region in the respective focal plane within the first photopolymerizable liquid and/or the further photopolymerizable liquid.

8. Method according to any one of the preceding claims, **characterized in that** at least the first polymerized structure in the first layer is irradiated from two directions by the first light radiation.

9. Method according to any one of the preceding claims, **characterized in that** the first light radiation and/or the further light radiation has a wavelength ranging from 200 to 1000 nm.

10. Method according to any one of the preceding claims, **characterized in that** the first light radiation and/or the further light radiation has a wavelength ranging from 380 to 780 nm.

11. Method according to any one of the preceding claims, **characterized in that**, during the creation of the three-dimensional multi-cell object, at least one element is integrated into the three-dimensional multi-cell object, said element being selected from the group consisting of membranes, channels, pores, sensors, electrically conductive carriers and chemotactic preparations.

12. Apparatus for creating a three-dimensional multi-cell object from photopolymerizable liquids, comprising
• a reaction vessel (4),
• a light source (1, 2), disposed in such a way that it can radiate light in a focal plane into the reaction vessel (4) during operation of the apparatus,
• a reservoir (7) for different photopolymerizable liquids,
• a pump (9), which can be flow connected to the reservoir (7) and the reaction vessel (4) in order to introduce the various photopolymerizable liquids into the reaction vessel (4) and to remove these from said reaction vessel, and
• a control unit (13) for controlling the light source (1, 2) and the pump (9),
**characterized in that**
the control unit is configured
- to control the light source in a first polymerization step to radiate light onto a first focal plane, which is located within a region of the reaction vessel (4) filled with the first liquid, in order to create a first polymerizable structure;
- to introduce a further photopolymerizable liquid into the reaction vessel by driving the pump,
- to control the light source in a further, second polymerization step in such a way that light radiates onto regions within a focal plane on which no light was radiated within the same focal plane in the first polymerization step for the purposes of creating a further polymerized structure in a further layer, wherein the further polymerized structure is disposed directly on the previously created first polymerized structure and connected to the latter, and wherein more than one polymerized structure is created in the same layer.

13. Apparatus according to Claim 12, **characterized in that** the light source (1, 2) is provided and configured to emit light of different wavelengths, wherein the wavelength of the light to be emitted can be specified.

14. Use of an apparatus according to Claim 12 or 13 for creating an artificial organ or a pregnancy model.

## Revendications

1. Procédé de production d'un objet multicellulaire en trois dimensions, avec les étapes suivantes:
a) introduire un premier liquide photopolymérisable dans une cuve de réaction (4),
b) focaliser un premier rayonnement lumineux sur un premier plan focal situé à l'intérieur d'une zone de la cuve de réaction (4), remplie du premier liquide,
c) produire une première structure polymérisée dans une première couche dans la cuve de réaction (4) par le premier rayonnement lumineux,
d) introduire un liquide photopolymérisable supplémentaire dans la cuve de réaction (4) de sorte qu'une structure polymérisée préalablement produite est recouverte au moins en partie du liquide photopolymérisable supplémentaire,
e) focaliser un rayonnement lumineux supplémentaire sur un plan focal supplémentaire situé à l'intérieur d'une zone de la cuve de réaction (4), remplie du liquide supplémentaire,
f) produire une structure polymérisée supplémentaire dans une couche supplémentaire dans la cuve de réaction (4) par le rayonnement lumineux supplémentaire, la structure polymérisée supplémentaire étant disposée directement sur la structure polymérisée préalablement produite et reliée à celle-ci,
g) répéter les étapes d) à f) avec un liquide photopolymérisable supplémentaire respectivement jusqu'à ce que l'objet multicellulaire en trois dimensions soit produit,
dans lequel le premier liquide photopolymérisable et/ou au moins l'un des liquides photopolymérisables supplémentaires contiennent des cellules biologiques,
**caractérisé en ce que** plus d'une structure polymérisée est produite dans la même couche.

2. Procédé selon la revendication 1, **caractérisé en ce que** le liquide photopolymérisable et au moins l'un des liquides photopolymérisables supplémentaires sont des liquides différents.

3. Procédé selon la revendication 1 ou 2, **caractérisé en ce que** le liquide photopolymérisable supplémentaire n'est introduit dans la cuve de réaction (4) que lorsque le liquide photopolymérisable se trouvant dans la cuve de réaction auparavant est retiré de la cuve de réaction (4) .

4. Procédé selon l'une quelconque des revendications précédentes, **caractérisé en ce que** dans la cuve de réaction (4) est disposée une plaque de support (15) à laquelle est reliée la première structure polymérisée.

5. Procédé selon l'une quelconque des revendications précédentes, **caractérisé en ce qu'**une refocalisation dans un système optique (5) qui est disposé entre une source de lumière (2) pour produire le premier rayonnement lumineux et/ou le rayonnement lumineux supplémentaire et la cuve de réaction (4) est effectuée pour modifier le plan focal à l'intérieur de la cuve de réaction (4).

6. Procédé selon l'une quelconque des revendications précédentes, **caractérisé en ce qu'**un mouvement relatif entre la cuve de réaction (4) ou une plaque de support (15) disposée dans la cuve de réaction (4) d'une part et une source de lumière (1, 2) pour produire le premier rayonnement lumineux et/ou le rayonnement lumineux supplémentaire d'autre part est effectué pour modifier le plan focal.

7. Procédé selon l'une quelconque des revendications précédentes, **caractérisé en ce que** le premier rayonnement lumineux et/ou le rayonnement lumineux supplémentaire sont dirigés sur une zone définie ou pouvant être prédéterminée dans le plan focal respectif à l'intérieur du premier liquide photopolymérisable et/ou du liquide photopolymérisable supplémentaire.

8. Procédé selon l'une quelconque des revendications précédentes, **caractérisé en ce qu'**au moins la première structure polymérisée est irradiée par le premier rayonnement lumineux dans la première couche à partir de deux directions.

9. Procédé selon l'une quelconque des revendications précédentes, **caractérisé en ce que** le premier rayonnement lumineux et/ou le rayonnement lumineux supplémentaire présentent une longueur d'onde dans la plage de 200 à 1000 nm.

10. Procédé selon l'une quelconque des revendications précédentes, **caractérisé en ce que** le premier rayonnement lumineux et/ou le rayonnement lumineux supplémentaire présentent une longueur d'onde dans la plage de 380 à 780 nm.

11. Procédé selon l'une quelconque des revendications précédentes, **caractérisé en ce que** pendant la production de l'objet multicellulaire en trois dimensions, on intègre dans l'objet multicellulaire en trois dimensions au moins un élément qui est sélectionné dans le groupe composé de membranes, de canaux, de pores, de capteurs, de supports électroconducteurs et de préparations chimiotactiques.

12. Dispositif de production d'un objet multicellulaire en trois dimensions dans un liquide photopolymérisable, comprenant
• une cuve de réaction (4),
• une source de lumière (1, 2) disposée de telle sorte qu'en cours de fonctionnement du dispositif, elle peut injecter de la lumière dans un plan focal dans la cuve de réaction (4),
• un réservoir (7) pour différents liquides photopolymérisables,
• une pompe (9) pouvant être mise en communication fluidique avec le réservoir (7) et la cuve de réaction (4) afin d'introduire les différents liquides photopolymérisables dans la cuve de réaction (4) et de les sortir de la cuve de réaction, et
• une unité de commande (13) pour commander la source de lumière (1, 2) et la pompe (9),
**caractérisé en ce que** l'unité de commande est configurée pour
- commander la source de lumière dans une première étape de polymérisation afin d'irradier de la lumière sur un premier plan focal situé à l'intérieur d'une zone de la cuve de réaction (4), remplie du premier liquide, afin de produire une première structure polymérisable ;
- introduire un liquide photopolymérisable supplémentaire dans la cuve de réaction par pilotage de la pompe,
- commander la source de lumière dans une deuxième étape de polymérisation supplémentaire de telle sorte que la lumière irradie des zones à l'intérieur d'un plan focal sur lesquelles aucune lumière n'a été irradiée à la première étape de polymérisation dans le même plan focal afin de produire une structure polymérisée supplémentaire dans une couche supplémentaire, la structure polymérisée supplémentaire étant disposée directement sur la première structure polymérisée produite préalablement et reliée à celle-ci, et plus d'une structure polymérisée étant produite dans la même couche.

13. Dispositif selon la revendication 12, **caractérisé en ce que** la source de lumière (1, 2) est prévue et aménagée pour émettre de la lumière de différentes longueurs d'onde, la longueur d'onde de la lumière à émettre pouvant être prédéterminée.

14. Utilisation d'un dispositif selon la revendication 12 ou 13 pour produire un organe artificiel ou un modèle de grossesse.
